(19)

Europäisches
Patentamt
European
Patent Office
Office européen
des brevets

(11) **EP 1 862 765 A1**

(12) **EUROPEAN PATENT APPLICATION**

(43) Date of publication:
 **05.12.2007 Bulletin 2007/49**

(51) Int Cl.:
 **G01C 22/00** (2006.01)

(21) Application number: **07108712.6**

(22) Date of filing: **23.05.2007**

(84) Designated Contracting States:
 **AT BE BG CH CY CZ DE DK EE ES FI FR GB GR
 HU IE IS IT LI LT LU LV MC MT NL PL PT RO SE
 SI SK TR**
 Designated Extension States:
 **AL BA HR MK YU**

(30) Priority: **29.05.2006 FI 20065359**

(71) Applicant: **Polar Electro Oy
 90440 Kempele (FI)**

(72) Inventors:
 • **Niemimäki, Mika
 90830, Haukipudas (FI)**
 • **Niva, Arto
 90940, Jääli (FI)**

(74) Representative: **Antila, Harri Jukka Tapani
 Kolster Oy Ab,
 Iso Roobertinkatu 23,
 (P.O. Box 148)
 00120 Helsinki (FI)**

(54) **Method and wrist device**

(57) The invention relates to a wrist device and to a method of determining movement information. The method comprises measuring acceleration from a movement of a wrist device worn by a user during the user's stepping; and determining movement pulses associated with a lateral movement generated by the user's stepping by using said acceleration.

Fig. 1

EP 1 862 765 A1

**Description**

FIELD

The invention relates to a method of determining movement information and to a wrist device.

BACKGROUND

There are different manners of measuring the step frequency associated with the progressive movement achieved by a person's stepping. Known measuring methods include pedometers attachable to the pelvis or footwear and based on mechanical pendulums or acceleration sensors and measuring acceleration in one or more directions. Techniques also exist for determining movement magnetically by utilizing the earth's magnetic field.

Drawbacks in prior art solutions are caused by the complexity of the movements generated by a person's stepping and problems caused by the complexity of movements in the determination of step frequency. Accordingly, it is useful to inspect techniques for determining movement information.

BRIEF DESCRIPTION

The object of the invention is to provide a wrist device and a method so as to achieve a reliable determination of a user's movement information. A first aspect of the invention is to provide a wrist device comprising a movement pulse determination unit for measuring acceleration from a movement of the wrist device during a user's stepping, the movement pulse determination unit being configured to determine movement pulses associated with a lateral movement generated by the user's stepping by using said acceleration.

A second aspect of the invention is to provide a method comprising measuring acceleration from a movement of a wrist device worn by a user during the user's stepping; and determining movement pulses associated with a lateral movement generated by the user's stepping by using said acceleration.

Preferred embodiments of the invention are described in the dependent claims.

The invention is based on determining movement pulses associated with a lateral movement generated by a user's stepping. When the user steps, the user's centre of gravity moves laterally as the user's bearing foot changes, each of the user's steps being associated with a change in the lateral movement of the centre of gravity. This causes a period in the user's lateral movement that corresponds to the step pair frequency. The user's upper extremities follow the movement of the centre of gravity, whereby the same period occurs in the lateral movement of the upper extremities, enabling the measurement of the movement pulses associated with the lateral movement with a wrist device.

The wrist device and method of the invention bring forth a plurality of advantages. The movement pulses associated with the lateral movement generated by stepping correlate well with the step frequency, the number and amplitude of extra movement pulses being slight. Accordingly, the use of the lateral movement generated by stepping provides a reliable result in the determination of movement pulses and derived information obtained from the movement pulses.

LIST OF FIGURES

In the following, the invention will be described in more detail in connection with preferred embodiments with reference to the accompanying drawings, in which

Figure 1 shows an example of a user's stepping dynamics;
Figure 2 shows a first example of the structure of a wrist device;
Figure 3 shows a second example of the structure of a wrist device;
Figure 4 shows a first example of measurement geometry;
Figure 5 shows a second example of measurement geometry;
Figure 6 shows a third example of the structure of a wrist device; and
Figure 7 shows a third example of measurement geometry;
Figure 8 shows an example of the structure of movement pulses;
Figure 9 shows a first example of a method in accordance with an embodiment of the invention; and
Figure 10 shows a second example of a method in accordance with an embodiment of the invention.

DESCRIPTION OF EMBODIMENTS

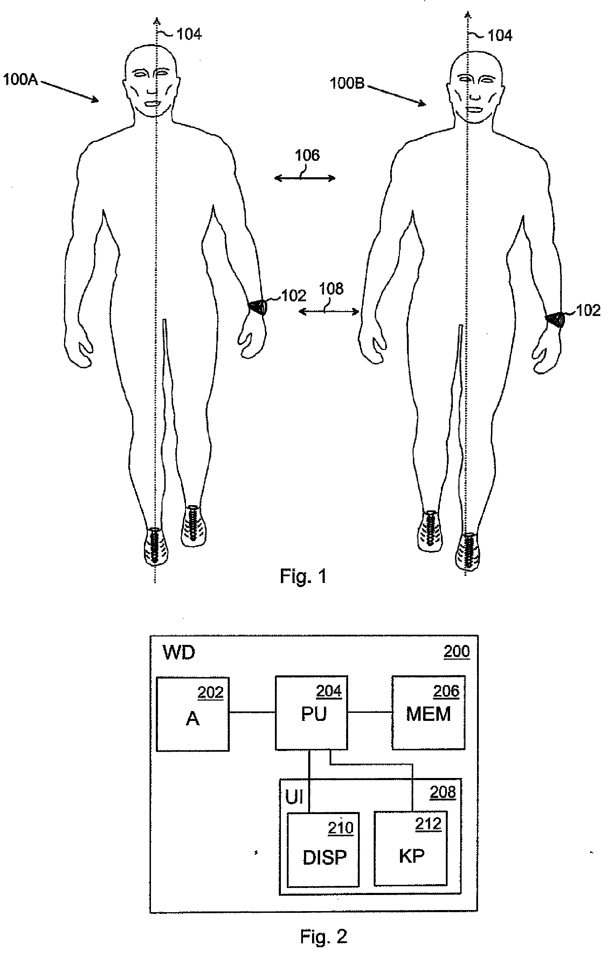

With reference to Figure 1, let us study the dynamics of the stepping of a user of a wrist device 102, wherein the user switches the centre of gravity from one foot to another. In situation 100A, the user leans on his/her right foot, and in situation 100B the user leans on his/her left foot. Progressive movement, such as walking or running, may be associated with stepping. Stepping may also take place in a state of suspended animation without progressive movement.

Between stepping steps 100A and 100B, the user's centre of gravity 104 shifts laterally in order for the user to maintain balance. Hereby, a lateral movement 106 of the centre of gravity 104 is generated by the stepping. When a plurality of successive shifts occurs in a user's stepping, the lateral movement 106 becomes periodic. A periodic lateral movement 106 may be interpreted as movement pulses.

A lateral movement 108 of the wrist device 102 is also associated with the lateral movement 106 of the centre of gravity 104. Accordingly, the lateral movement 106 of the centre of gravity 104 may be characterized by studying the lateral movement 108 of the wrist device, and determine the movement pulses generated by the stepping.

Figure 2 shows an example of the structure of a wrist device 200. The wrist device 200 typically comprises a processing unit (PU) 204, a memory unit (MEM) 206, an acceleration sensor (A) 202, and a user interface (UI) 208.

The processing unit 204 may be implemented by using analog circuits, ASIC circuits (Application Specific Integrated Circuit), a digital processor, a memory and computer software. The processing unit 204 may constitute part of the computer of the wrist device 200. The processing unit 204 may execute a computer process according to encoded instructions stored in the memory unit 206 for determining movement information.

In an embodiment, the acceleration sensor 202 is based on piezo technology (piezoresistor). In piezoresistor technology, a material is used whose resistance changes as the material is compressed. Mass acceleration generates a force directed to the piezoresistor, and when constant current is led through the piezoresistor, the current acting over the piezoresistor changes according to the compression caused by acceleration.

In piezoelectric technology, a piezoelectric sensor generates the resistance when the acceleration sensor is accelerated.

In silicon bridge technology, a silicon chip is etched such that silicon mass remains on the silicon chip at the end of the silicon beam. When acceleration is directed to the silicon chip, the silicon mass directs a force to the silicon beam, changing the resistance of the silicon beam.

Micro-machined silicon technology is based on the use of a differential capacitor. Voice coil technology is based on the same principle as a microphone. Some examples of suitable movement sensors include: Analog Devices ADXL105, Pewatron HW or VTI Technologies SCA series.

The acceleration sensor 202 may also be based on other technologies suitable for the purpose, for example a gyroscope integrated into a silicon chip, a micro-vibration placed in a panel mounting component or a mechanical pendulum.

Acceleration information generated by the acceleration sensor 202 may be transferred to the processing unit 204 or to the memory unit 206.

The user interface 208 typically comprises a display unit (DISP) 210 and a display controller. The display unit 210 may comprise LCD components (Liquid Crystal Display), for example. The display unit 210 may graphically and/or numerically display, to the user, a movement pulse accumulation or a secondary parameter value, such as the number of steps, the distance progressed or the energy consumption, determined from movement pulses characterizing the performance.

The user interface 124 may further comprise a keypad (KP) 212 allowing the user to input commands in the wrist device 200.

In an embodiment, the wrist device 200 is a pulse counter, in which case the wrist device 200 may comprise a receiver for receiving a signal transmitted from a pulse measurement unit. The pulse measurement unit may be a belt-like structure installed on the user's chest and comprising means for performing an electrocardiogram measurement (ECG) and for transmitting ECG information to the wrist device 200.

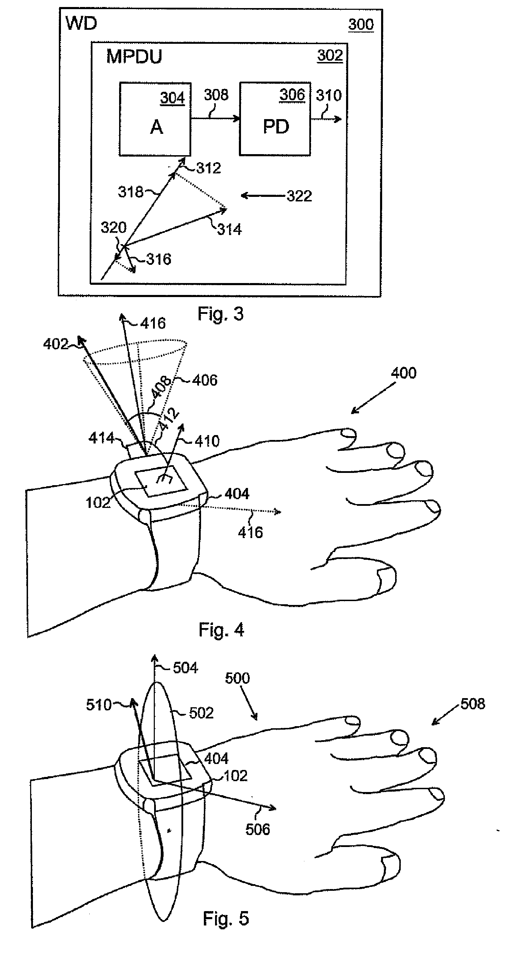

With reference to Figure 3, a wrist device (WD) 300 comprises a movement pulse determination unit 302 for measuring acceleration from the movement of the wrist device 300 during the stepping of the user of the wrist device 300. The movement pulse determination unit 302 determines movement pulses associated with the lateral movement 108 generated by the user's stepping by the use of acceleration.

The movement determination unit 302 typically comprises an acceleration sensor (A) 304 and a pulse detector (PD) 306.

The acceleration sensor 304 determines instantaneous acceleration values and generates a data stream 308 characterizing the instantaneous acceleration values. The acceleration sensor 304 feeds the data stream 308 into the pulse detector 306.

The pulse detector 306 receives the data stream 308 and indicates acceleration variations associated with the lateral movement 108 from the data stream 306. The pulse detector 306 may calculate the pulses it identifies and output pulse information 310 for processing or storage.

The pulse detector 306 may be implemented for instance by means of computer software executed by means of the processing unit 204 according to Figure 2 and stored in the memory unit 206.

In an embodiment of the invention, the acceleration sensor 304 measures acceleration in the direction of the lateral movement 108 generated by the user's stepping. The pulse detector 306 determines movement pulses associated with the user's lateral movement 108 by using said acceleration in the direction of the lateral movement 108.

The acceleration sensor 304 may be a one-dimensional acceleration sensor 304 for measuring acceleration in the direction of the lateral movement generated by stepping. Figure 3 shows a vector diagram 322 comprising a measuring direction 312 of the one-dimensional acceleration sensor, acceleration 314 in the direction of the lateral movement 108 of the wrist device, and acceleration 316 perpendicular to the lateral movement 108. The acceleration 316 perpendicular to the lateral movement 108 may be caused by an upturned movement of the wrist device 300 generated in stepping or a movement in the direction of the user's progressive movement.

The one-dimensional acceleration sensor 302 measures projections of mutually orthogonal accelerations in the measuring direction 312. The projection of the acceleration 314 in the direction of the lateral movement 108 is shown by vector 318, and the projection of the acceleration 316 perpendicular to the lateral movement 108 is shown by a vector 320.

In an embodiment of the invention, the one-dimensional acceleration sensor 302 is oriented in the wrist device such that the projection of the acceleration 314 in the direction of the lateral movement 108 dominates with respect to the projections 320 of the accelerations 316 perpendicular to the lateral movement 108.

The orientation of the one-dimensional acceleration sensor 302 also takes account of the orientation of the acceleration sensor 304 with respect to the body of the wrist device 300, the orientation of the wrist device 300 with respect to the user's upper extremity, and the user's stepping style. The stepping style affects the way the reference part, such as the back of the hand or the carpal vertebra, of the user's upper extremity, moves during stepping.

With reference to the example of Figure 4, in an embodiment of the invention, the one-dimensional acceleration sensor 304 is oriented such that the measuring direction 402 is restricted with respect to a normal vector 410 of the user's back of hand 400 at an angle of 45 degrees, and inside a cone 406 generated around the vector 416 perpendicular to the fingers, the spread angle 408 of the cone 406 being previously known. The spread angle 408 is 90 degrees, for example. In this case, the measuring distance 402 compensates for the differences occurring in the different users' stepping styles, the movements of the extremities and the installation of the wrist device 102. The normal vector 410 may also be determined as a normal vector starting from the plane 404 of the wrist device 102.

In the example of Figure 4, the angle 412 is 45 degrees, and the angle 414 is a right angle. The vector 416 determining the direction of the cone 406 may also be directed to the opposite side of the wrist device 404 in accordance with Figure 4.

The plane 404 of the wrist device may be determined for instance as the plane of the glass of the wrist device 102 or as a support plane of the wrist device 102 against the wrist. The plane 404 of the wrist device 102 may also be an imaginary plane.

With reference to Figure 5, in an embodiment, the one-dimensional acceleration sensor 304 is oriented to measure acceleration whose main component 510 is, during the use of the wrist device, at a plane determined by the normal vector 504 of the user's back of hand 500 and a vector 506 perpendicular with respect to the normal vector of the user's fingers 508 and back of hand 500. The normal vector 504 may also be determined from the plane 404 of the wrist device 102, and the plane 502 is an imaginary plane generated in a wristband-like manner around the user's wrist.

In an embodiment, the main measuring direction of the one-dimensional acceleration sensor 304 is substantially in the direction of the normal vector 504 of the plane 404 of the wrist device.

In an embodiment, the main measuring direction of the one-dimensional acceleration sensor 304 is at the plane 404 of the wrist device, and when the wrist device 500 is in use, in the direction of a vector, such as vector 510, perpendicular to the user's fingers.

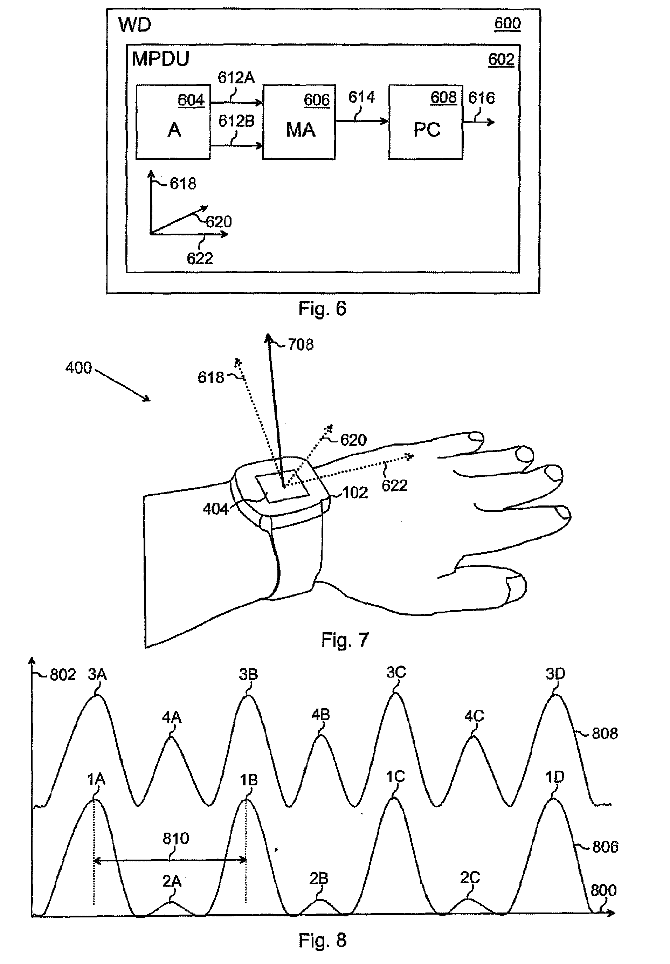

With reference to Figures 6 and 7, the movement pulse determination unit 602 of the wrist device 600 measures a plurality of directional acceleration components 618, 620, 622 and determines acceleration 708 in the direction of the lateral movement generated by the user's stepping by combining the plurality of directional acceleration components 618 to 622. In a vector presentation, acceleration $a_S$ in the direction of the lateral movement may be presented in the form:

$$a_S = a_1 \hat{a}_1 + a_2 \hat{a}_3 + a_3 \hat{a}_3, \qquad (1)$$

wherein $a_1, a_2$ and $a_3$ are linear coefficients and $\hat{a}_1, \hat{a}_2$ and $\hat{a}_3$ are directional acceleration components 618 to 622. The linear coefficients $a_1, a_2$ and $a_3$ may be selected such that a linear combination presents acceleration optimally in the direction of the lateral movement 108. The linear coefficients $a_1, a_2$ and $a_3$ may be coefficients determined in the manufacturing stage of the wrist device 600 or they may be determined during use in the wrist device 600. The linear coefficients may be calculated in the processing unit 204, for example.

The wrist device 600 may comprise a multidimensional acceleration measurement unit (AMU) 604, which measures acceleration in a plurality of directions. The measurement geometry is typically well determined, allowing a deterministic resultant acceleration to be calculated from the accelerations measured in the different directions. The measuring elements measuring the acceleration in different directions may be mutually perpendicular or a prearranged angle may exist therebetween. The acceleration measurement unit 604 may be implemented by means of a multidimensional acceleration sensor or a plurality of one-dimensional acceleration sensors. The acceleration measurement unit 604 may additionally comprise a movement analyzer 606 for determining the linear combination of the directional acceleration components 618 to 622.

The movement analyzer 606 receives signals 612A, 612B, which characterize acceleration components, from the acceleration measurement unit 604 and determines the linear coefficients of the acceleration signals, for example, by utilizing the pulse structure generated by the linear combination. The movement analyzer 606 may calculate the linear combination and feed the linear combination to the pulse detector 608, which determines movement pulses associated with the lateral movement 108 by using said linear combination 614 of the directional components. The pulse information 616 may be further led to processing or for display to a user.

The movement analyzer 606 may be implemented by means of the processing unit 204 as a computer process, for example.

In Figure 8, an example of the structure of a movement pulse is studied. The horizontal axis 800 shows time in a random unit, and the vertical axis 802 shows the strength of the movement pulse in an acceleration unit, for example. The first curve 808 represents a situation wherein the movement pulses are determined in a random direction, such as in the longitudinal direction of the user's wrist. Pulses 3A to 3D characterize step pair frequency, and interference pulses 4A to 4C are generated by the reciprocal movement of the hands in the direction of travel, for example. In the situation of curve 808, the interference pulses 4A to 4C may be interpreted as pulses characterizing the step pair frequency in pulse identification, whereby an erroneous step pair frequency is obtained as the result. In this situation, the amplitude of the interference pulses 4A to 4C maybe very different for different users, and, accordingly, the identification of the pulses 3A to 3D may be uncertain.

The second curve 806 represents a situation wherein the direction of the acceleration sensors is optimized for the measurement of the lateral movement 108, and movement pulses 1A to 1 D characterize the lateral movement. Interference pulses 2A to 2C are generated by a slightly erroneous orientation of the acceleration sensor. In this situation, the amplitude of the interference pulses 2A to 2C is significantly less than in the case of curve 808, and an erroneous interpretation of the movement pulses is significantly less likely. In addition, the user-dependence of the amplitudes of the interference pulses 2A to 2C is slight. The step pair frequency is obtained by determining a time interval 810 of two successive movement pulses and by taking the inverse value from the time interval.

The movement pulses determined by the invention may be used for a plurality of purposes. The processing unit 204 may calculate the step pair frequency, the velocity, the path travelled and/or the energy consumption, for example, from the movement pulses.

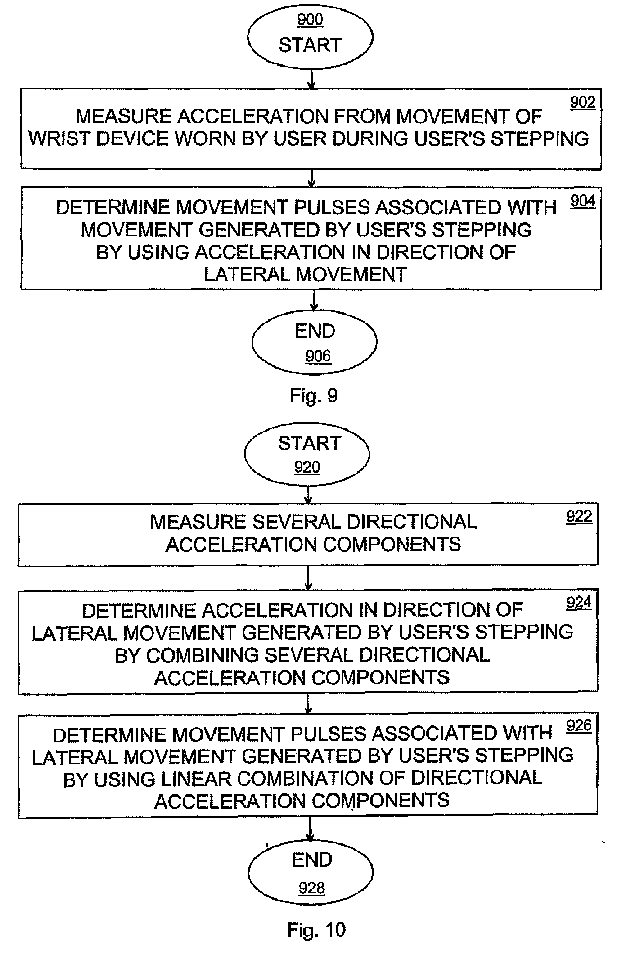

With reference to Figures 9 and 10, let us study methods according to embodiments of the invention.

With reference to Figure 9, the method starts at 900.

At 902, acceleration is measured from the movement of the wrist device worn by the user during the user's stepping.

At 904, movement pulses associated with the lateral movement 108 generated by the user's stepping are determined by using said acceleration.

In an embodiment, at 902, acceleration in the direction of the lateral movement 108 generated by the user's stepping is measured, and, at 904, movement pulses associated with the lateral movement 108 generated by the user's stepping are determined by using said acceleration in the direction of the lateral movement.

In an embodiment, acceleration in the direction of the lateral movement generated by the user's stepping is measured at 902 with a one-dimensional acceleration sensor 304.

In an embodiment, at 902, acceleration in the direction of the lateral movement 108 generated by the user's stepping is measured with the one-dimensional acceleration sensor 304, whose measurement direction is restricted relative to the normal vector of the user's back of hand at a 45-degree angle and inside a cone generated around a vector perpendicular to the fingers, the spread angle of the cone being previously known.

In an embodiment, at 902, acceleration in the direction of the lateral movement generated by the user's stepping is measured with the one-dimensional sensor 304, the main component of whose acceleration is at a plane determined by the normal vector of the user's back of hand and a vector perpendicular with respect to the normal vector of the user's fingers and back of hand.

The method ends at 906.

With reference to Figure 10, the method starts at 920.

At 922, several directional acceleration components 618 to 622 are measured.

At 924, a linear combination of the directional acceleration components 618 to 622 is determined, the combination being in the direction of the lateral movement 108 generated by the user's stepping.

At 926, movement pulses associated with the lateral movement 108 generated by the user's stepping are determined by using several directional components 618 to 622, such as the linear combination of the directional components 618 to 622, for example.

The method ends at 928.

The method may be implemented as a computer process to be stored in the memory unit 206 and to be executed in the processing unit 205, for example. The computer process may be included in encoded instructions that may be implemented with some known programming language. The encoded instructions maybe included in a computer software product whose physical expression may be a memory means or a signal. The memory means may be an optical or magnetic memory data entry device, for example.

Although the invention is described above with reference to the example in accordance with the accompanying drawings, it will be appreciated that the invention is not to be so limited, but may be modified in a variety of ways within the scope of the appended claims.

**Claims**

1. A wrist device, c h a r a c t e r i z e d in that the wrist device comprises:

   a movement pulse determination unit (302) for measuring acceleration from a movement of the wrist device during a user's stepping, the movement pulse determination unit (302) being configured to determine movement pulses associated with a lateral movement generated by the user's stepping by using said acceleration.

2. A wrist device as claimed in claim 1, wherein the movement pulse determination unit (302) is configured to measure acceleration in the direction of the lateral movement generated by the user's stepping and to determine movement pulses associated with the lateral movement generated by the user's stepping by using said acceleration in the direction of the lateral movement.

3. A wrist device as claimed in claim 2, wherein the movement pulse determination unit (302) comprises a one-dimensional acceleration sensor (304) configured to measure acceleration in the lateral movement generated by stepping.

4. A wrist device as claimed in claim 3, wherein the one-dimensional acceleration sensor (304) is configured to measure acceleration in the measuring direction, the measuring direction being restricted with respect to a normal vector of the user's back of hand at an angle of 45 degrees, and inside a cone generated around a vector perpendicular to the fingers, the spread angle of the cone being previously known.

5. A wrist device as claimed in claim 3, wherein the one-dimensional acceleration sensor (304) is configured to measure acceleration in the measuring direction, the main component of the acceleration being, during the use of the wrist device, at a plane determined by a normal vector of the user's back of hand and a vector perpendicular with respect to the normal vector of the user's fingers and back of hand.

6. A wrist device as claimed in claim 1, wherein the movement pulse determination unit (302) is configured to measure a plurality of directional acceleration components and to determine acceleration in the direction of the lateral movement generated by the user's stepping by combining a plurality of directional acceleration components.

7. A wrist device as claimed in claim 6, wherein the movement pulse determination unit (602) comprises:

   a multidimensional acceleration measurement unit (604) configured to measure a plurality of directional acceleration components;
   a movement analyzer (606) for determining a linear combination of the directional acceleration components, the linear combination of the directional acceleration components being in the direction of the lateral movement generated by the user's stepping; and
   the movement pulse determination unit (602) is configured to determine movement components associated with the lateral movement generated by the user's stepping by using said linear combination of the directional components.

8. A method of determining movement information, **characterized by** the method comprising:

measuring (902) acceleration from a movement of a wrist device worn by a user during the user's stepping; and determining (904) movement pulses associated with a lateral movement generated by the user's stepping by using said acceleration.

9. A method as claimed in claim 8, further comprising:

measuring (902) acceleration in the direction of the lateral movement generated by the user's stepping; and determining (904) movement pulses associated with the lateral movement generated by the user's stepping by using said acceleration in the direction of the lateral movement.

10. A method as claimed in claim 8, further comprising measuring (902) acceleration in the direction of the lateral movement generated by the user's stepping with a one-dimensional acceleration sensor.

11. A method as claimed in claim 8, further comprising measuring (902) acceleration in the direction of the lateral movement generated by the user's stepping with a one-dimensional acceleration sensor, the measuring direction of which is restricted with respect to a normal vector of the user's back of hand at an angle of 45 degrees, and inside a cone generated around a vector perpendicular to the fingers, the spread angle of the cone being previously known.

12. A method as claimed in claim 8, further comprising measuring (902) acceleration in the direction of the lateral movement generated by the user's stepping with a one-dimensional acceleration sensor, the main component of whose acceleration is, during the use of the wrist device, at a plane determined by a normal vector of the user's back of hand and a vector perpendicular with respect to the normal vector of the user's fingers and back of hand.

13. A method as claimed in claim 8, further comprising:

measuring (922) a plurality of directional acceleration components; and determining (926) acceleration the direction of the lateral movement generated by the user's stepping by combining a plurality of directional acceleration components.

14. A method as claimed in claim 13, further comprising measuring (922) a plurality of directional acceleration components; and

determining (924) a linear combination of the directional acceleration components in the direction of the lateral movement generated by the user's stepping; and determining (926) movement pulses associated with the lateral movement generated by the user's stepping by using said linear combination of directional components.

15. A computer program comprising encoded instructions for executing a computer process of claim 8.

16. A computer program product comprising a computer program of claim 15.

17. A memory means comprising a computer program of claim 15.

18. A signal comprising a computer program of claim 15.

Fig. 1

Fig. 2

WD 300

MPDU 302

304
A

308

306
PD

310

312
318
322
320
316
314

Fig. 3

416
402
406
408
412
414
410
102
404
416

Fig. 4

504
510
502
500
508
404
102
506

Fig. 5

## Fig. 6

WD     <u>600</u>

MPDU     <u>602</u>

<u>604</u> A    612A / 612B    <u>606</u> MA    614    <u>608</u> PC    616

618    620    622

**Fig. 6**

## Fig. 7

400

708

618

620

622

102

404

**Fig. 7**

## Fig. 8

802   3A   3B   3C   3D

4A   4B   4C   808

1A   1B   1C   1D

810   806

2A   2B   2C   800

**Fig. 8**

900
START

902
MEASURE ACCELERATION FROM MOVEMENT OF
WRIST DEVICE WORN BY USER DURING USER'S STEPPING

904
DETERMINE MOVEMENT PULSES ASSOCIATED WITH
MOVEMENT GENERATED BY USER'S STEPPING
BY USING ACCELERATION IN DIRECTION OF
LATERAL MOVEMENT

END
906

Fig. 9

START
920

922
MEASURE SEVERAL DIRECTIONAL
ACCELERATION COMPONENTS

924
DETERMINE ACCELERATION IN DIRECTION OF
LATERAL MOVEMENT GENERATED BY USER'S STEPPING
BY COMBINING SEVERAL DIRECTIONAL
ACCELERATION COMPONENTS

926
DETERMINE MOVEMENT PULSES ASSOCIATED WITH
LATERAL MOVEMENT GENERATED BY USER'S STEPPING
BY USING LINEAR COMBINATION OF DIRECTIONAL
ACCELERATION COMPONENTS

END
928

Fig. 10

European Patent Office

**EUROPEAN SEARCH REPORT**

Application Number

EP 07 10 8712

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2004/186695 A1 (AOSHIMA ICHIRO [JP] ET AL) 23 September 2004 (2004-09-23) | 1-3,5-18 | INV. G01C22/00 |
| X | * page 10, paragraphs 51,52; figures 29,31 * | 4,11 | |
| | ----- | | |
| X | EP 1 253 404 A (LADETTO QUENTIN [CH]; GABAGLIO VINCENT [BE]; VAN SEETERS JOSEPHUS [US]) 30 October 2002 (2002-10-30) * page 3, paragraph 20 * | 1-18 | |
| | ----- | | |
| X | US 2005/240375 A1 (SUGAI YOSHINORI [JP]) 27 October 2005 (2005-10-27) * page 3, paragraph 45; figure 5 * | 1-3,8-10 | |
| | ----- | | |
| A | EP 1 199 544 A1 (OMRON TATEISI ELECTRONICS CO [JP] OMRON HEALTHCARE CO LTD [JP]) 24 April 2002 (2002-04-24) * column 12, paragraph 47; figures 20a,c * | 1-18 | |
| | ----- | | TECHNICAL FIELDS SEARCHED (IPC) |
| A | US 2002/152645 A1 (DARLEY JESSE [US] ET AL) 24 October 2002 (2002-10-24) * page 2, paragraph 19; figure 8 * | 1-18 | G01C |
| | ----- | | |

The present search report has been drawn up for all claims

| Place of search | Date of completion of the search | Examiner |
|---|---|---|
| The Hague | 13 September 2007 | Dragomir, Adrian |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

EP 1 862 765 A1

## ANNEX TO THE EUROPEAN SEARCH REPORT
## ON EUROPEAN PATENT APPLICATION NO.

EP 07 10 8712

This annex lists the patent family members relating to the patent documents cited in the above-mentioned European search report.
The members are as contained in the European Patent Office EDP file on
The European Patent Office is in no way liable for these particulars which are merely given for the purpose of information.

13-09-2007

| Patent document cited in search report | | | Publication date | Patent family member(s) | | | Publication date |
|---|---|---|---|---|---|---|---|
| US 2004186695 | A1 | | 23-09-2004 | CN | 1530880 | A | 22-09-2004 |
| | | | | JP | 3801163 | B2 | 26-07-2006 |
| | | | | JP | 2004290658 | A | 21-10-2004 |
| EP 1253404 | A | | 30-10-2002 | AU | 781848 | B2 | 16-06-2005 |
| | | | | AU | 3561502 | A | 24-10-2002 |
| | | | | CA | 2382999 | A1 | 23-10-2002 |
| | | | | US | 2003018430 | A1 | 23-01-2003 |
| US 2005240375 | A1 | | 27-10-2005 | CN | 1690660 | A | 02-11-2005 |
| | | | | GB | 2413633 | A | 02-11-2005 |
| | | | | JP | 2005309693 | A | 04-11-2005 |
| EP 1199544 | A1 | | 24-04-2002 | DE | 60119058 | T2 | 07-12-2006 |
| | | | | JP | 3543778 | B2 | 21-07-2004 |
| | | | | JP | 2002191580 | A | 09-07-2002 |
| | | | | US | 2002089425 | A1 | 11-07-2002 |
| US 2002152645 | A1 | | 24-10-2002 | NONE | | | |

EPO FORM P0459

For more details about this annex : see Official Journal of the European Patent Office, No. 12/82

13